# EUROPEAN PATENT SPECIFICATION

(11) **EP 1 259 136 B1**
(45) Date of publication and mention of the grant of the patent: **24.03.2004**
(21) Application number: 01900552.9
(22) Date of filing: 16.01.2001
(51) Int. Cl.: A45D 34/00, A45D 40/00, A45D 33/02

(54) **FRAGRANCE DISPENSING DEVICE**
PARFUMSPENDER
DISPOSITIF DE DIFFUSION DE PARFUM

(30) Priority: 17.01.2000 GB 0001038
(43) Date of publication of application: 27.11.2002
(73) Proprietor: The Aroma Company (Europe) Limited, Wallingford, Oxfordshire OX10 0BG (GB)
(72) Inventor: HARROP, Simon, Wallingford Oxfordshire OX10 0BG (GB); GOODWIN, David, Pyrford Surrey GU22 8XD (GB)
(74) Representative: Rees, David Christopher
(86) International application number: PCT/GB2001/000163
(87) International publication number: WO 2001/052693

(56) References cited:
- FR-A- 936 227
- FR-A- 2 511 856
- US-A- 1 852 685
- US-A- 5 018 894

## Description

The present invention relates to a fragrance dispensing device. In particular, the invention relates to a device for allowing a user to sample the fragrance of consumable products such as cleaning, cosmetic, toiletry, food or beverage products.

Previous examples of dispensing devices include a device for dispensing perfumes that includes a fan to propel the perfume towards a user. Although successful in some applications, this and other conventional devices have several disadvantages. For example, these devices are complex owing to the need for a fan and associated driving means. The fan requires maintenance and eventually replacement due to wear. In addition, the fan creates an undesirable noise and restricts the design (such as the size) of other components of the device. The driving means requires energy input, such as a battery, that needs periodic replacement and is environmentally unattractive.

A dispenser according to the preamble of claim 1 is known from FR-A-2511856.

The aim of the present invention is at least to alleviate some of the disadvantages associated with the prior art. The aim includes the provision of a simpler and more efficient device useful for a wider range of applications.

According to the present invention there is provided a fragrance dispensing device comprising an enclosure; valve means; a fragrance absorbent within the enclosure; an outlet from the enclosure; and means for expelling air from the enclosure through the outlet, together with fragrance liberated from the absorbent, the means for expelling air comprising a manually operable bellows, characterised in that when the bellows are depressed, the valve means is arranged to open in a first direction for air to be expelled from the enclosure and when the bellows is released the valve means is arranged to open in a second direction for air to enter the enclosure.

This device offers several advantages including its surprising simplicity of construction and minimisation of parts which do not compromise its efficiency. This simplicity reduces the cost of manufacture, the labour and skill required for maintenance and repairs, and increases its durability. No electrical means are required, so the apparatus offers the advantage of being environmentally attractive. The operation is manual, and the fragrance may be liberated by operation of the bellows and subsequent air flow and/or diffusion. The valve means gives the advantage that the air in the enclosure is prevented from leaking out except upon demand (i.e. when the bellows are depressed). The useful lifetime of the apparatus is therefore maximised. A valve so specified is a simple and economical way of offering these advantages. In addition, there is no noise created by any driving means as in the prior art. The apparatus is suitable for a range of applications including user sampling for purchase consideration or testing, owing in part to its simplistic construction. This simplicity also facilitates the ease of replacing any of the component parts. In one embodiment, the outlet is a hole on the first inclined part of the bellows. In another embodiment, the aperture faces upwards.

Preferably, the bellows is releasably connectable to the enclosure. This offers the advantage that the bellows can be simply and quickly removed and replaced when required. The bellows may form part of the enclosure, may be connected to the enclosure or may actually constitute the enclosure. These features further add to the simplicity of the device. In one embodiment, the bellows includes a bayonet end for connection to the enclosure. In other embodiments, connection is by way of sticky tape, a clamp, glue or friction holding.

Preferably, the fragrance absorbent is within the bellows. One advantage of this is that the bellows can be easily removed as a single unit and replaced when the absorbent needs replenishing.

The bellows may be blow moulded. In other embodiments, other methods of manufacture that are suitable can be employed. In a typical embodiment the bellows are of a plastics material, although other materials such as metal can be used.

Preferably, the absorbent is exfoliated vermiculite granules, a felt pad, or a gel; although any other material can be used that can hold and retain a volume of fragrance typically in a liquid form. In one embodiment, the absorbent is fibrous, while in another it is granular. In one embodiment in which the absorbent is vermiculite granules, the granules can be held in a sachet-made of a suitable material such as a non-woven synthetic material such as Aerotex ^{RTM}. The absorbent is suitable for absorbing a wide range of fragrances.

The fragrance may be a liquid fragrance of a cosmetic, cleaning, toiletry, food or beverage product. The toiletry may be a lotion or a deodorant; the cosmetic may be a perfume; the food or beverage may be pizza or a soft drink, respectively. In an alternative embodiment, the product may be children's modelling material. An advantage of the present invention is that it is suitable for use with a diversity of consumable products.

Preferably, the outlet is sufficiently small so that air and fragrance are expelled from the enclosure as a jet. This offers the advantage that air and fragrance can be directed towards a user's nose.

The valve means may comprise a first valve arranged to open in the first direction, and a second valve arranged to open in the second direction, and preferably constitutes an inlet valve and an outlet valve. The provision of two such one-way valves, or in other embodiments, similar such features, provides the advantages described while at the same time being consistent with the aim of a simple apparatus construction.

There may be further included a fascia detachably positionable in proximity to the bellows means. In one embodiment, the fascia is attached by glue onto the enclosure. In other embodiments, a clamp, sticky tape, a weld, clip or friction holds the fascia in its relative position. The fascia offers the advantage of being a convenient means by which a display can be positioned close to the bellows means. In an embodiment of this invention, the fascia is a customised vacuum formed and printed trim fascia, although other suitable methods of manufacture can be used. Information, such as branding or product details may be printed on the fascia, or displayed on the fascia in some way.

Preferably, the fascia, includes a recess; and there is further included a recess cover pivotable about an upper or a lower edge of the recess. The cover can therefore be pivotable about an uppermost recess edge for either vertical orientation of the fascia. In one embodiment, the recess cover is a clear plastic window to hold the price/product information in place. This advantageously enables the retailer to retain the electronic point of sale (EPOS) ticket which would otherwise be obscured by the device when attached to a ticket strip on a shelf edge.

Preferably, the enclosure is injection moulded, although in other embodiments other methods of manufacture such as blow moulding are used. Typically, the enclosure comprises a plastics material or a metal.

The enclosure may be formed integrally with a shelf or other shelving means, or may include some form of attachment for attaching the enclosure to a shelf. In one embodiment, the rear of the enclosure includes lugs arranged to grip a shelf edge. This gives a positive location, and prevents the unit moving backwards as it is depressed. Other bodies than shelves may be preferred, for example, a shipper tray, a retail shelf, vacuum formed tray, promotional bin, cardboard formed gondola end, or other point of sale found in a supermarket, retailer, or public area. Alternatively, the shelving means may comprise a display for testing or consumer trials, a vending machine, or an advertising holding. The attaching means may include a clip, clamp, double-sided tape or other fastening device.

The enclosure is preferably located substantially above or below the component to which it is attached.

Preferably, the bellows comprise the enclosure. This has the advantage of reducing yet further the complexity of the device.

Preferably, there is further included a collar mountable on the bellows as a bayonet fit. The bayonet collar enables the bellows to be easily attached to a point of sale, or used for interactive smell sampling in museums or visitor centres, for example.

In such a case, the bellows may be used in combination with a compressing device, such as a gate-folded card. In a first configuration of the card, the bellows is compressed substantially flat by the compressing means device, while in a second configuration, the bellows is uncompressed. This offers the advantage that the bellows can be easily posted, or inserted into another device when substantially flat and then later used when uncompressed. The compressing means thereby provides a simple way of enabling the bellows to be conveniently stored or transported before use.

In other embodiments, other arrangements can be used, or other such materials such as plastics. The compressing means may be placed in some other packaging device, such as a wallet or envelope.

The present invention also therefore extends to an envelope including the device. The invention may be carried into practice in various ways, and embodiments will now be described by way of example with reference to the accompanying drawings in which:
Fig. 1 is an exploded side view in part section of the components of a first embodiment of the present invention;
Fig. 2 is a part sectional side view of the components of Fig. 1 as assembled;
Fig. 3 is a front view of the components of Fig. 1 as assembled;
Fig. 4a is a part sectional exploded side view of a bellows with a cap;
Fig. 4b is a part sectional exploded side view of a bellows with a bayonet collar, and a cap;
Fig. 5a is a part sectional side view of the cap;
Fig. 5b is a front view of the cap;
Fig. 5c is a side view of an alternative cap;
Fig. 6 is a front view of a facia;
Fig. 7a is a sectional front view of an aperture in the facia;
Fig. 7b is a part sectional front view of the aperture in the main body;
Fig. 8a is a sectional side view of a main body;
Fig. 8b is a sectional side view of an alternative main body;
Figs. 9a and 9b are part sectional side views of the device of the first embodiment of the present invention in relation to its shelf; and
Figs, 10a-10c are side views of a second embodiment of the device of the present invention in three different configurations.

The present invention relates to a fragrance dispensing device for use, for example, by a user for sampling a fragrance of a consumable product by pressing an identified area on the device. This pressing action causes a pair of bellows to depress, and expel air from the device through an outlet. The device can therefore be used to aid a user in deciding whether to purchase the product, or consider the fragrance for testing purposes, for example.

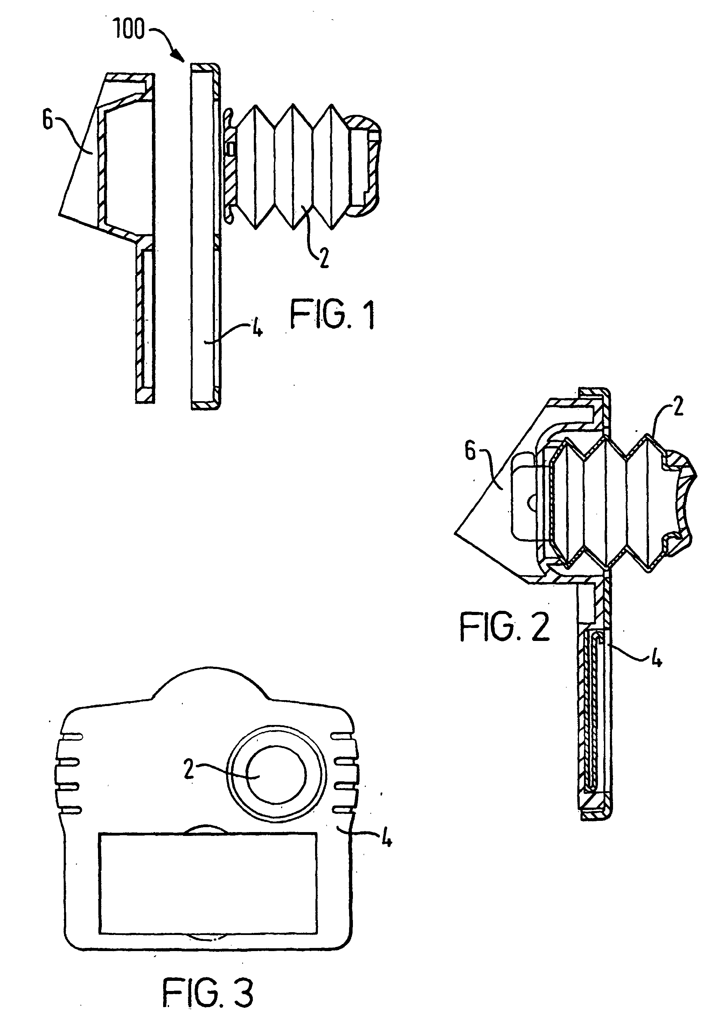

Figure 1 is an exploded side view in part-section of the components of a fragrance dispensing device 100, which comprises an enclosure, according to a first embodiment of the present invention. The main components comprise a pair of manually operable bellows 2, a trim fascia 4, and a main body 6.

Figure 2 is a part-sectional side view of the components of Figure 1 as assembled. The bellows 2 attach to the main body 6 by a bayonet fitting. The main body 6 is attached to the fascia by glue, for example.

Figure 3 is a front view of the components of Figure 2.

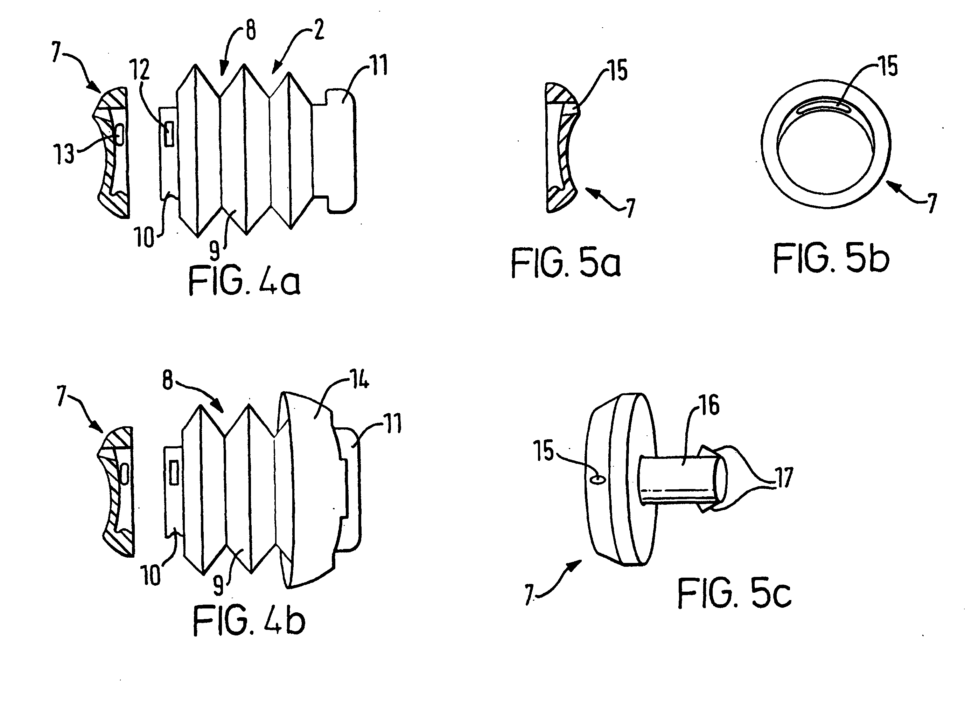

Figures 4a and 5 illustrate the bellows 2 in further detail. The bellows are blow moulded from a flexible plastics material. They are approximately 3 cm in length. The bellows comprises main body 8 and an end cap 7. The body 8 takes the form of a convoluted portion 9 with a collar 10 at one end which is open and a bayonet fitting 11 at the other end, which is closed. Figure 4b shows an alternative bellows 2, in which the closed end includes a bayonet collar 14. The collar 14 slides over the bayonet end 11 of the bellows 2, being attached thereto as a bayonet fit by a quarter turn. The collar 10 carries a stud 12. The cap 7 is generally shaped as a hollow button with an opening 13; when the cap 7 and body 8 are assembled, the cap 7 fits over the collar 10 and the stud 12 engages the opening 13 thereby ensuring the correct relative orientation. The bayonet fitting 11 engages the main body 6.

In the present embodiment, the bellows 2 are filled through the open end with an absorbent (not shown). The absorbent is a fragrance absorbable porous support medium comprising granules of exfoliated vermiculite. The granules are placed loose inside the bellows, although in another embodiment they may be placed in a sachet with a sealed edge. The granules are soaked in a liquid fragrance. The fragrance is that of the fragrance formulation that is found incorporated into the original consumable product.

Figure 5a is a part-sectional side of the cap 7. A hole 15 is included in the cap 7 through which air can pass into and out of the bellows 2. In another embodiment, one or more holes would be included on the first inclined surface of the bellows 2. The hole 15 faces upwards irrespective of the orientation of the device. Alternatively, in another embodiment a hole may be positioned behind the front of the first end of the bellows 2, and a channel would run from behind the bellows 2 to another point where air is expelled towards to the user. Figure 5b shows a front view of the cap 7 and air hole 15. Figure 5c shows an alternative to the cap 7 in side view. The cap 7 includes a hollow projection 16 for receipt in the collar 10. The projection 16 includes a pair of lugs 17 giving a positive fix when inserted into the collar 10, preventing the cap being pulled off.

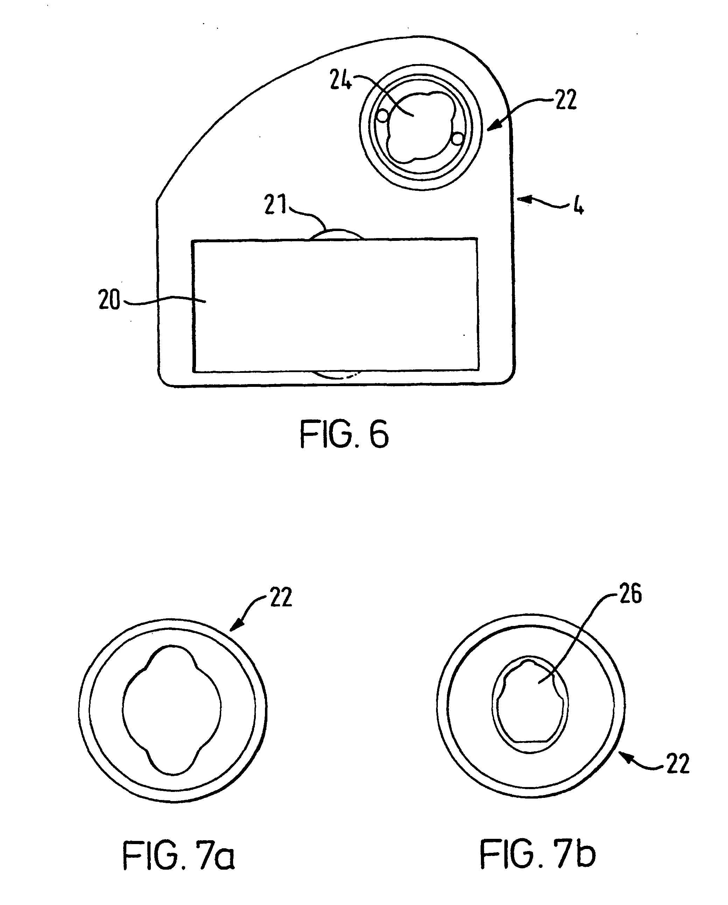

The trim fascia 4 is shown in a front view in Figure 6. The fascia 4 is a vacuum formed and printed trim fascia. A recess 20 is included for product branding and/or pricing information. The recess 20 has a recess cover (not shown) comprising a clear plastic window to hold product and/or price information in place. The clear plastic window can be hinged to the uppermost horizontal edge defining the recess for either vertical orientation of the trim fascia 4, i.e. the window is 'reversible'. Finger cut outs 21 are provided for both options. The fascia is approximately 10 cm square in size.

The fascia 4 includes an aperture 22 through which the bellows can be inserted. The aperture 22 includes a quarter-turn bayonet hole for insertion of the bellows 2 as shown in Figure 7a. Figure 7b is a front part-sectional view of the aperture. An oval hole 26 is included to ensure that the cap is correctly orientated.

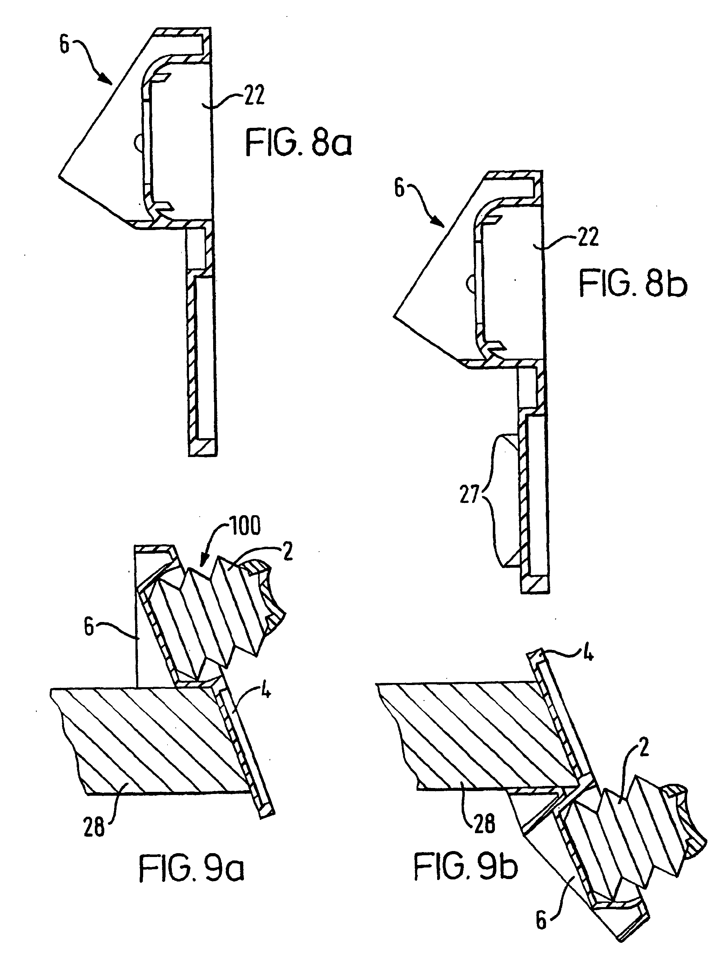

The main body is illustrated in sectional side view in Figure 8a. The main body is injection moulded from a plastics material and is substantially of the same height as the fascia 4.

Figure 8b shows an alternative main body. Four legs 27, or small teeth (two on each side) are provided at the rear of the main body to grip a shelf edge.

Figures 9a and 9b illustrate the assembled components of the device 100 in relation to a shelf 28. The main body 6 includes attaching means such as double-sided adhesive tape (not shown) for attachment to the shelf 28 as shown in Figure 9a, which is a part-sectional side view of the device 100. In this Figure, the main body 6 is attached to the shelf 28 so that it (and the fragrance product branding information) sit substantially above the bulk of the shelf 28.

Figure 9b shows the device 100 in which an alternative to the main body 6 is adapted to sit substantially below the bulk of the shelf by reversing the body of the device 100. The reversibility of the clear plastic window enables it to be opened at the bottom (being hinged at the top) when the main body is fixed substantially above or below the shelf.

In use, the absorbent (not shown) is soaked in the fragrance of the consumable product to be dispensed, and then placed inside the bellows 2 at the time of assembly. The components of the device 100 are assembled, with the bellows 2 being positioned through the aperture 22 in the fascia 4. The main body 6 is then attached to the shelf 28.

To use the device, the user presses the end of the bellows 2 showing through the fascia, which in appearance resembles a button. Pressing this button causes the bellows 2 to depress, and thence air from inside the bellows together with fragrance liberated from the absorbent is expelled through the hole 15. The user will then smell not the actual product, but air fragranced by the same fragrance formulation incorporated into the original product.

Typically, the fragrance will be replenished or replaced periodically with, for example, the apparatus being disposed of, recycled or part-replaced. Usually, the main body will be saved and newly charged bellows 2 and/or new facsia 4 with different information used.

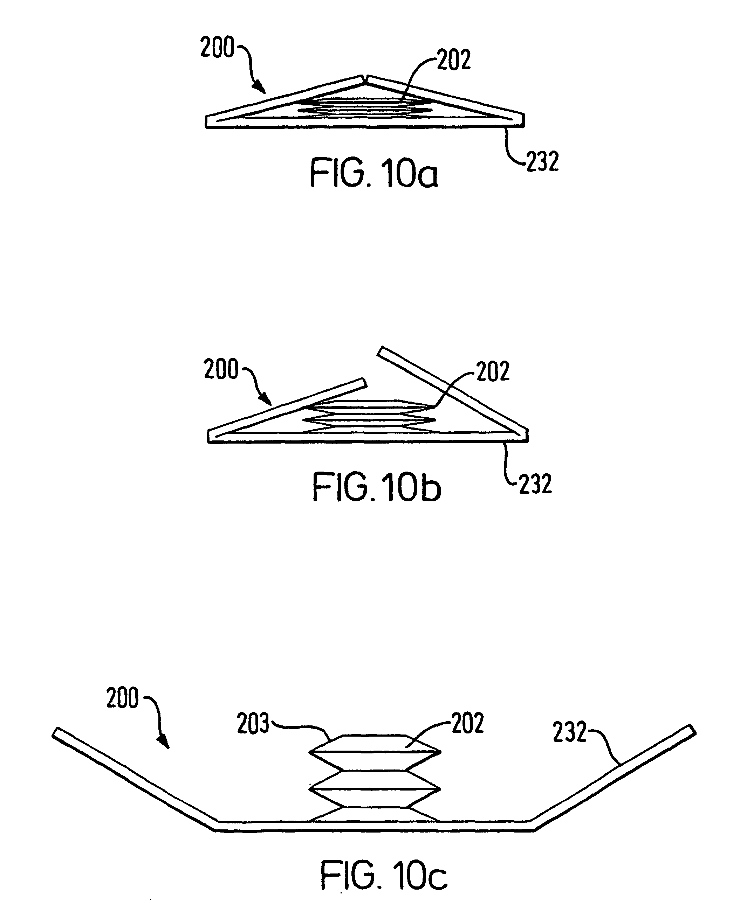

Figures 10a to 10c show a side view of the device 200 of a second embodiment of the present invention. Similar reference numerals have been used to describe similar features.

The bellows 202 are attached to a compressing means 232 in the form of a gate-folded piece of card. The card 232 is adapted to compress the bellows substantially flat when in a closed configuration, as shown in Figure 10a. Figure 10b shows the card 232 part-open, with the bellows part-compressed. The decompression of the bellows 202 may be instigated by taking the card out of an envelope, for example, in which the card has been inserted.

Figure 10c shows the card 232 fully open and the bellows 202 fully released.

In use, once the card has been opened, the bellows 202 are ready for depression as described in relation to the first embodiment. Fragranced air will be liberated from inside the bellows 202 through an outlet (not shown). The outlet would probably be most conveniently located in the first inclined surface 203 of the bellows 202.

## Claims

1. A fragrance dispensing device (100, 200) comprising an enclosure; a fragrance absorbent within the enclosure; an outlet (15) from the enclosure; and means for expelling air from the enclosure through the outlet (15), together with fragrance liberated from the absorbent, the means for expelling air comprising a manually operable bellows (2, 202) **characterised in that** when the bellows (2, 202) is depressed, a valve means is arranged to open in a first direction for air to be expelled from the enclosure and when the bellows is released said valve means is arranged to open in a second direction for air to enter the enclosure.

2. A device as claimed in claim 1 in which the bellows (2, 202) is releasably connectable to the enclosure.

3. A device as claimed in claim 2 in which the bellows connects to the enclosure as a bayonet fit (11).

4. A device as claimed in claim 1 in which the bellows (2, 202) forms part of the enclosure.

5. A device as claimed in any one of the preceding claims in which the fragrance absorbent is within the bellows (2, 202).

6. A device as claimed in any one of the preceding claims in which the bellows (2, 202) is of blow moulded construction.

7. A device as claimed in any one of the preceding claims in which the absorbent is exfoliated vermiculite granules, a felt pad, or a gel.

8. A device as claimed in any one of the preceding claims in which the fragrance is a liquid fragrance of a cosmetic, cleaning, toiletry, food or beverage product.

9. A device as claimed in any one of the preceding claims in which the outlet is sufficiently small so that air and fragrance are expelled from the enclosure as a jet.

10. A device as claimed in claim 1 in which the valve means comprises a first valve arranged to open in the first direction, and a second valve arranged to open in the second direction.

11. A device as claimed in any one of the preceding claims further including a fascia (4) detachably positionable in proximity to the bellows (2, 202).

12. A device as claimed in claim 11 in which the fascia (4) includes a recess; and in which there is further included a recess cover pivotable about an upper or a lower edge of the recess.

13. A device as claimed in any one of the preceding claims in which the enclosure is injection moulded.

14. A device as claimed in any one of the preceding claims in which the enclosure is formed integrally with a shelving means.

15. A device as claimed in any one of claims 1 to 13 in which the enclosure includes attaching means for attaching the enclosure to a shelving means.

16. A device as claimed in any one of the preceding claims in which the enclosure is arranged to be located substantially above or below a shelving means, in use.

17. A device as claimed in any one of the preceding claims in which the bellows (2, 202) comprises the enclosure.

18. A device as claimed in claim 15, when dependent upon claim 3, further including a collar mountable on the bellows (2, 202) as a bayonet fit.

19. A device as claimed in claim 17 in combination with a compressing means; and in which in a first configuration the bellows means (2, 202) is compressed substantially flat by the compressing means, and in a second configuration the bellows means is uncompressed.

20. A combination as claimed in claim 19 in which the compressing means is a gate-folded card.

21. A combination as claimed in claim 19 or claim 17 located within an envelope.

## Patentansprüche

1. Parfumspendervorrichtung (100, 200), welche eine Kapselung, ein Parfumabsorptionsmittel innerhalb der Kapselung, einen Auslass (15) aus der Kapselung und Mittel aufweist, um Luft gemeinsam mit Aromastoff, das aus dem Absorptionsmittel freigesetzt wird, durch den Auslass (15) aus der Kapselung auszustoßen, wobei die Mittel zum Ausstoßen von Luft einen manuell bedienbaren Balg (2, 202) aufweisen, **dadurch gekennzeichnet, dass** - wenn der Balg (2, 202) gedrückt wird - ein Ventilorgan so angeordnet ist, dass es sich in eine erste Richtung öffnet, damit Luft aus der Umschließung ausgestoßen wird, und dadurch, dass - wenn der Balg losgelassen wird - das Ventilorgan so angeordnet ist, dass es sich in eine zweite Richtung öffnet, damit Luft in die Kapselung eintritt.

2. Vorrichtung nach Anspruch 1, bei welcher der Balg (2, 202) mit der Kapselung lösbar verbindbar ist.

3. Vorrichtung nach Anspruch 2, bei welcher der Balg mit der Kapselung mittels Bajonettverschluss (11) verbunden ist.

4. Vorrichtung nach Anspruch 1, bei welcher der Balg (2, 202) Teil der Kapselung ist.

5. Vorrichtung nach einem der vorhergehenden Ansprüche, bei welcher das Aromaabsorptionsmittel sich innerhalb des Balgs (2, 202) befindet.

6. Vorrichtung nach einem der vorhergehenden Ansprüche, bei welcher der Balg (2, 202) aus einer blasgeformten Konstruktion besteht.

7. Vorrichtung nach einem der vorhergehenden Ansprüche, bei welcher das Absorptionsmittel geschälte Vermiculitgranulat, ein Filzkissen oder ein Gel ist.

8. Vorrichtung nach einem der vorhergehenden Ansprüche, bei welcher der Aromastoff ein flüssiges Aroma eines Kosmetik-, Reinigungs-, Toiletten-, Nahrungsoder Getränkeproduktes ist.

9. Vorrichtung nach einem der vorhergehenden Ansprüche, bei welcher der Auslass ausreichend klein ist, so dass Luft und Aromastoff als Strahl aus der Kapselung ausgestoßen werden.

10. Vorrichtung nach Anspruch 1, bei welcher das Ventilorgan ein erstes Ventil, das angeordnet ist, um sich in der ersten Richtung zu öffnen, und ein zweites Ventil aufweist, das angeordnet ist, um sich in die zweite Richtung zu öffnen.

11. Vorrichtung nach einem der vorhergehenden Ansprüche, welche ferner ein Sims (4) aufweist, das abnehmbar in der Nähe des Balgs (2, 202) angeordnet werden kann.

12. Vorrichtung nach Anspruch 11, in der das Sims (4) eine Vertiefung enthält und in der ferner eine Vertiefungsabdeckung enthalten ist, welche um eine obere oder eine untere Kante der Vertiefung drehbar ist.

13. Vorrichtung nach einem der vorhergehenden Ansprüche, bei welcher die Kapselung spritzgegossen ist.

14. Vorrichtung nach einem der vorhergehenden Ansprüche, bei welcher die Kapselung mit einem Lagerungsorgan einstückig ausgebildet ist.

15. Vorrichtung nach einem der Ansprüche 1 bis 13, bei welcher die Kapselung Befestigungsmittel aufweist, um die Kapselung an einem Lagerungsorgan zu befestigen.

16. Vorrichtung nach einem der vorhergehenden Ansprüche, bei welcher die Kapselung so angeordnet ist, dass sie sich im Gebrauch im Wesentlichen oberhalb oder unterhalb eines Lagerungsorgans befindet.

17. Vorrichtung nach einem der vorhergehenden Ansprüche, bei welcher der Balg (2, 202) die Kapselung enthält.

18. Vorrichtung nach Anspruch 15, wenn von Anspruch 3 abhängig, bei welcher die Vorrichtung ferner einen Kragen aufweist, der auf dem Balg (2, 202) als Bajonettverschluss befestigbar ist.

19. Vorrichtung nach Anspruch 17 in Kombination mit einem Komprimiermittel, und bei welcher in einer ersten Konfiguration das Balgmittel (2, 202) durch das Komprimiermittel im Wesentlichen flach gedrückt ist und in einer zweiten Konfiguration das Balgmittel nicht zusammengedrückt ist.

20. Kombination nach Anspruch 19, bei welcher das Komprimiermittel eine gittertorartig faltbare Karte ist.

21. Kombination nach Anspruch 19 oder 17, welche sich innerhalb einer Hülle befindet.

## Revendications

1. Dispositif de diffusion de parfum (100, 200) comprenant un boîtier; un absorbant de parfum à l'intérieur du boîtier; un orifice de sortie (15) du boîtier; et un moyen destiné à expulser l'air du boîtier par l'orifice de sortie (15), conjointement avec du parfum libéré par l'absorbant, le moyen destiné à expulser l'air comprenant un soufflet (2, 202) pouvant fonctionner manuellement **caractérisé en ce que**, lorsqu'on appuie sur le soufflet (2, 202), un moyen de soupape est agencé pour s'ouvrir dans une première direction afin que l'air soit expulsé du boîtier et, lorsque le soufflet est relâché, ledit moyen de soupape est agencé pour s'ouvrir dans une seconde direction afin que l'air entre dans le boîtier.

2. Dispositif selon la revendication 1 dans lequel le soufflet (2, 202) peut être raccordé au boîtier de façon à pouvoir se relâcher.

3. Dispositif selon la revendication 2 dans lequel le soufflet est raccordé au boîtier par emboîtement à baïonnette (11).

4. Dispositif selon la revendication 1 dans lequel le soufflet (2, 202) forme une partie du boîtier.

5. Dispositif selon l'une quelconque des revendications précédentes dans lequel l'absorbant de parfum se trouve dans le soufflet (2, 202).

6. Dispositif selon l'une quelconque des revendications précédentes dans lequel le soufflet (2, 202) est de construction moulée par soufflage.

7. Dispositif selon l'une quelconque des revendications précédentes dans lequel l'absorbant est constitué de grains de vermiculite exfoliée, d'un coussinet de feutre ou d'un gel.

8. Dispositif selon l'une quelconque des revendications précédentes dans lequel le parfum est un parfum liquide d'un produit cosmétique, de nettoyage, de toilette, alimentaire ou d'une boisson.

9. Dispositif selon l'une quelconque des revendications précédentes dans lequel l'orifice de sortie est suffisamment petit pour que l'air et le parfum soient expulsés du boîtier sous forme d'un jet.

10. Dispositif selon la revendication 1 dans lequel le moyen de soupape comprend une première soupape agencée pour s'ouvrir dans la première direction, et une seconde soupape agencée pour s'ouvrir dans la seconde direction.

11. Dispositif selon l'une quelconque des revendications précédentes comprenant en outre une enseigne (4) pouvant être positionnée de façon détachable à proximité du soufflet (2, 202).

12. Dispositif selon la revendication 11 dans lequel l'enseigne (4) comprend un évidement; et dans lequel est en outre compris un couvercle d'évidement pouvant pivoter autour d'un bord supérieur ou inférieur de l'évidement.

13. Dispositif selon l'une quelconque des revendications précédentes dans lequel le boîtier est moulé par injection.

14. Dispositif selon l'une quelconque des revendications précédentes dans lequel le boîtier est formé de manière intégrale avec un moyen de rayonnage.

15. Dispositif selon l'une quelconque des revendications 1 à 13 dans lequel le boîtier comprend un moyen de fixation destiné à fixer le boîtier à un moyen de rayonnage.

16. Dispositif selon l'une quelconque des revendications précédentes dans lequel le boîtier est agencé de façon à se situer sensiblement au-dessus ou en dessous d'un moyen de rayonnage, lors de l'utilisation.

17. Dispositif selon l'une quelconque des revendications précédentes dans lequel le soufflet (2, 202) comprend le boîtier.

18. Dispositif selon la revendication 15, si dépendante de la revendication 3, comprenant en outre un collier pouvant être monté sur le soufflet (2, 202) par emboîtement à baïonnette.

19. Dispositif selon la revendication 17 en association avec un moyen de compression; et dans lequel dans une première configuration le moyen de soufflet (2, 202) est comprimé sensiblement à plat par le moyen de compression, et dans une seconde configuration le moyen de soufflet est décompressé.

20. Association selon la revendication 19 dans laquelle le moyen de compression est une carte à volets.

21. Association selon la revendication 19 ou la revendication 17 située à l'intérieur d'une enveloppe.
